## Europäisches Patentamt

## European Patent Office

## Office européen des brevets

(19)

(11) Veröffentlichungsnummer: **0 212 225 B1**

(12)

# EUROPÄISCHE PATENTSCHRIFT

(45) Veröffentlichungstag der Patentschrift: 24.04.91

(21) Anmeldenummer: 86109553.7

(22) Anmeldetag: 12.07.86

(51) Int. Cl.5: **A61M 25/00, A61M 5/00**

(54) Vorrichtung zur Steuerung einer eine Ummantelung aufweisenden Sonde eines Endoskopiegeräts.

(30) Priorität: 24.07.85 DE 3526434

(43) Veröffentlichungstag der Anmeldung:
04.03.87 Patentblatt 87/10

(45) Bekanntmachung des Hinweises auf die
Patenterteilung:
24.04.91 Patentblatt 91/17

(84) Benannte Vertragsstaaten:
FR GB IT

(56) Entgegenhaltungen:
EP-A- 0 122 089
FR-A- 450 345
FR-A- 2 365 999
GB-A- 2 094 628

(73) Patentinhaber: Karl Storz GmbH & Co.
Mittelstrasse 8
W-7200 Tuttlingen(DE)

(72) Erfinder: Miketic, Sinisa, Dr. med.
Im Eichenhof 11b
W-3500 Kassel(DE)
Erfinder: Schade, Udo
Ochshäuser Strasse 52
W-3500 Kassel(DE)
Erfinder: Schade, Michael, Dipl.-Ökonom
Mangenberger Strasse 221
W-5650 Solingen(DE)

(74) Vertreter: Freiherr von Schorlemer, Reinfried,
Dipl.-Phys.
Patentanwalt Brüder-Grimm-Platz 4
W-3500 Kassel(DE)

## Beschreibung

Die Erfindung betrifft eine Vorrichtung nach dem Oberbegriff des Patentanspruchs 1. Eine derartige Vorrichtung ist aus FR-A-2 365 999 bekannt.

Die Anwendung bekannter Endoskope, insbesondere Fiberskope, ist mit beträchtlichem Aufwand und vielen Schwierigkeiten verbunden. Bei der endoskopischen Verödung von Speiseröhren-Krampfadern beispielsweise muß der das Endoskopiegerät betätigende Arzt mit einer Hand die Optik bedienen, mit der anderen Hand das Vorderende der Sonde positionieren und gleichzeitig mit seinen Augen die Positionierung beobachten und überwachen. Er benötigt daher eine Hilfsperson, die auf seinen Befehl hin die am einen Ende der Sonde befestigte Injektionsnadel vor- und zurückbewegt und eine mit dieser verbundene Injektionsspritze betätigt. Eine zweite Hilfsperson muß schließlich den die Sonde umgebenden Endoskopieschlauch in die Speiseröhre des Patienten einführen und ihn in dieser nach Angaben des Arztes hin- und herbewegen. Aufgrund der daraus resultierenden Koordinationsschwierigkeiten zwischen dem Arzt und den beiden Hilfspersonen ist die Verödung einer Krampfader zum Nachteil des Patienten häufig nicht in der gewünschten kurzen Zeit und mit der erforderlichen Präzision durchführbar. Darüber hinaus ist das Verfahren sehr personalaufwendig.

Entsprechende Probleme ergeben sich bei der Anwendung herkömmlicher Endoskopiegeräte für andere Zwecke und mit anderen Sonden, z.B. solchen, die anstelle der Injektionsnadel ein anderes medizinisches Instrument in Form eines Greifers, einer Koagulationsschlinge, einer Biopsiezange oder dergleichen aufweisen.

Der Erfindung liegt die Aufgabe zugrunde, die eingangs bezeichnete Vorrichtung derart auszubilden, daß der die Verödung durchführende Arzt die Steuerung der Sonde allein vornehmen kann, so daß dadurch nicht nur eine Hilfsperson eingespart, sondern auch eine wesentliche Verbesserung der Steuergenauigkeit erzielt wird.

Diese Aufgabe wird erfindungsgemäß mit den im Kennzeichen des Patentanspruchs 1 angegebenen Mitteln gelöst.

Die Erfindung bringt den Vorteil mit sich, daß die Steuerung der Sonde weitgehend automatisch vorgenommen werden kann. Der Arzt braucht lediglich die Schaltvorrichtung zu bedienen. Diese weist vorzugsweise vom Arzt leicht erreichbare Schalter, z.B. einen mit dem Fuß betätigbaren Doppelschalter auf, so daß sie vom Arzt betätigt werden kann, ohne daß dieser seine Hände vom Endoskopiegerät nehmen muß. Dadurch wird einerseits die die Sonde steuernde Hilfsperson überflüssig, andererseits der Steuervorgang erleichtert und präziser gestaltet.

Weitere vorteilhafte Merkmale der Erfindung ergeben sich aus den Unteransprüchen.

Die Erfindung wird nachfolgend in Verbindung mit der beiliegenden Zeichnung an zwei Ausführungsbeispielen näher erläutert. Es zeigen:

Fig. 1    die Draufsicht auf eine erfindungsgemäße Vorrichtung zur Steuerung der Injektionssonde eines Endoskopiegeräts;

Fig. 2    die Unteransicht eines beweglichen Schlittens der Vorrichtung nach Fig. 1;

Fig. 3    die Unteransicht der gesamten Vorrichtung nach Fig. 1;

Fig. 4    eine Schutzschaltung zur Steuerung des Ein- und Ausfahrens der Injektionsnadel der Sonde;

Fig. 5    eine Schaltung zur Steuerung von Kontrolleuchten, die das Ende des Bewegungshubs des Schlittens anzeigen;

Fig. 6    eine Schaltung zur Steuerung eines Schiebers für die Betätigung einer mit der Injektionssonde verbundenen Injektionsspritze; und

Fig. 7    die Draufsicht auf die Vorrichtung nach Fig. 1 bei Steuerung alternativer Endoskopiesonden.

Die erfindungsgemäße Vorrichtung zur Steuerung einer Injektionssonde 1 eines Endoskopiegeräts weist ein Gehäuse 2 auf, auf dessen Oberseite ein Schlitten 3 in Führungen 4 verschiebbar gelagert ist. Dabei ist der Schlitten 3 in Richtung eines Doppelpfeils v hin- und herverschiebbar. An einem vorderen Teil des Gehäuses 2 ist eine erste Aufnahme 5 befestigt. Diese dient zur beispielsweise klemmenden Aufnahme und Festlegung eines Teils einer Ummantelung 6 der Injektionssonde 1. Am Schlitten 3 ist eine mit der ersten Aufnahme 5 koaxiale zweite Aufnahme 7 befestigt, die zur beispielsweise klemmenden Aufnahme und Festlegung eines Bedienungsorgans 8 der Injektionssonde 1 dient. Die Ummantelung 6 der Injektionssonde 1 wird von einem Betätigungselement 9 durchsetzt, das einen aus dem einen Ende der Ummantelung 6 herausragenden, mit dem Bedienungsorgan 8 verbundenen Endabschnitt aufweist. Der andere Endabschnitt des Betätigungselements 9 trägt ein medizinisches Instrument in Form einer Injektionskanüle 10, die mittels des Betätigungselements 9 in das vordere Ende der Ummantelung 6 zurückgezogen bzw. aus diesem Ende nach außen vorgeschoben werden kann. Das Betätigungselement 9 wirkt nach Art eines Bowdenzugs, der mittels des Bedienungsorgans 8 betätigt wird, und ist in diesem Fall hohl. Das Bedienungsorgan 8 ist ebenfalls hohl und weist einen Anschluß für das verjüngte Ende eines Zylinders 11 auf, in dem ein Kolben 12 hin- und herverschiebbar gelagert ist, der zusammen mit dem Zylinder 11 eine medizinische Spritze darstellt. Zur Halterung der Spritze am Schlitten 3 weist dieser eine dritte, beispielsweise durch Klemmwirkung wirksame Aufnahme 13 auf, in der der Zylinder 11 festgelegt werden kann. Die Aufnahme 13 ist auf der von der ersten Aufnahme 3 abgewandten Seite der zweiten Aufnahme 7 angeordnet und erstreckt sich koaxial zu den Aufnahmen 5 und 7.

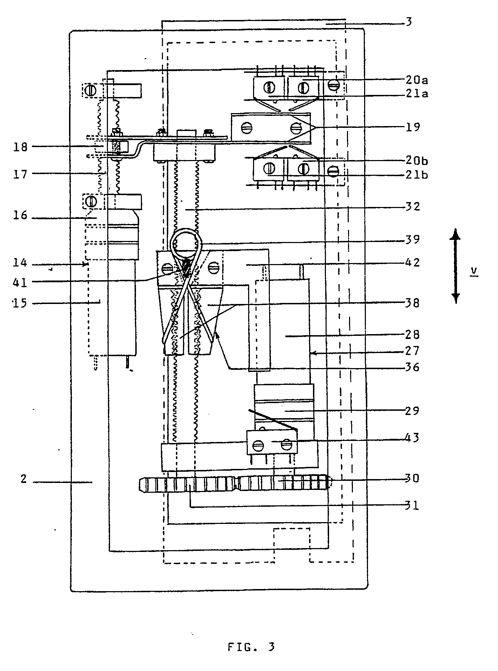

Gemäß Fig. 3 ist in einem unteren Teil des Gehäuses 2 eine erste Antriebseinheit 14 untergebracht. Diese weist einen am Gehäuse 2 befestigten Reversiermotor 15 auf, dessen Ausgangswelle über ein Reduziergetriebe 16 eine drehbar im Gehäuse gelagerte Gewindestange 17 in Umdrehungen versetzen kann. Auf die Gewindestange 17 ist eine Gewindemutter 18 aufgesetzt, die einen radial wegragenden, mit dem Schlitten 3 verbundenen Arm 19 aufweist. Dadurch wird die beim wahlweisen Einschalten des Reversiermotors 15 in der einen oder anderen Drehrichtung erzielte Drehbewegung der Gewindestange 17 in eine lineare, parallel zum Doppelpfeil v verlaufende Hin- und Herverschiebung des Schlittens 3 umgewandelt.

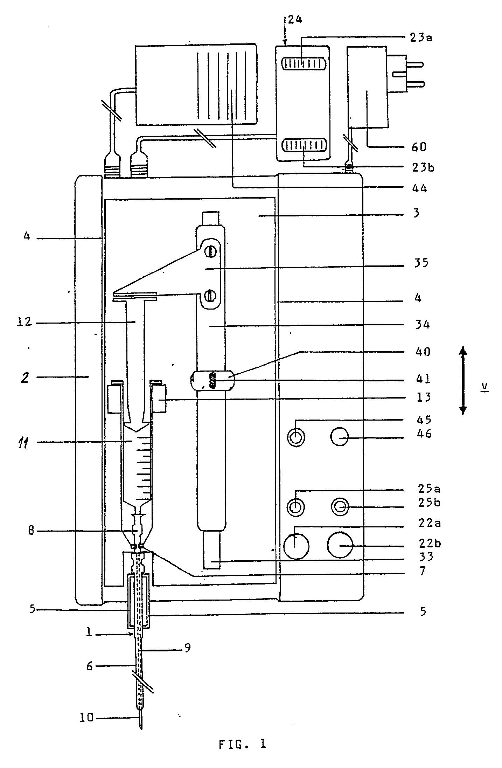

Der Arm 19 ist zwischen zwei in Richtung des Doppelpfeils v beabstandeten Paaren von Endschaltern 20a,b und 21a,b angeordnet. Die Endschalter 20a,b legen den Bewegungshub des Schlittens 3 in Richtung des Doppelpfeils v fest, indem sie so in einen Schaltkreis des Reversiermotors 15 geschaltet sind, daß dieser nach dem Einschalten automatisch jeweils ausgeschaltet und damit der Schlitten 3 angehalten wird, wenn der Arm 19 an einen der Endschalter 20a,b anschlägt. Dabei ist der Bewegungshub des Schlittens 3 so gewählt, daß er genau dem zum Aus- bzw. Einfahren der Injektionskanüle 10 benötigten maximalen Weg entspricht. Die entsprechend angeordneten Endschalter 21a,b sind je einer auf der Gehäuseoberfläche angeordneten Kontrolleute 22a,b (Fig. 1) zugeordnet, die aufleuchtet, sobald der Schlitten 3 seine entsprechende Endstellung erreicht hat. Nach Erreichen dieser Endstellung kann der Reversiermotor 15 jeweils in der entgegengesetzten Drehrichtung eingeschaltet werden.

Die beschriebene Vorrichtung ermöglicht nach dem Festlegen der Injektionssonde 1 in den Aufnahmen 5 und 7 ein motorisches Ein- und Ausfahren der Injektionskanüle 10 durch wahlweises Einschalten des Reversiermotors 15 in der einen oder anderen Drehrichtung. Um dem Arzt, der das im übrigen bekannte und daher nicht weiter dargestellte Endoskopiegerät bedient, die Möglichkeit zu geben, auch die notwendigen Einschaltungen des Reversiermotors 15 selbst vorzunehmen, sind in dessen Schaltkreis zweckmäßig zwei mit dem Fuß zu betätigende Schalter 23a,b geschaltet, die beispielsweise zu einem Doppelschalter 24 zusammengefaßt sind. Alternativ könnte es sich um Schalter handeln, die so am Endoskopiegerät angeordnet werden, daß sie vom Arzt leicht mit einem freien Finger od. dgl. erreicht werden können. Schließlich können in den Schaltkreis des Reversiermotors 15 noch zwei Handschalter 25a,b geschaltet sein, die dieselben Funktionen wie die Schalter 23a,b und damit vor Inbetriebnahme der Vorrichtung eine Funktionskontrolle gestatten.

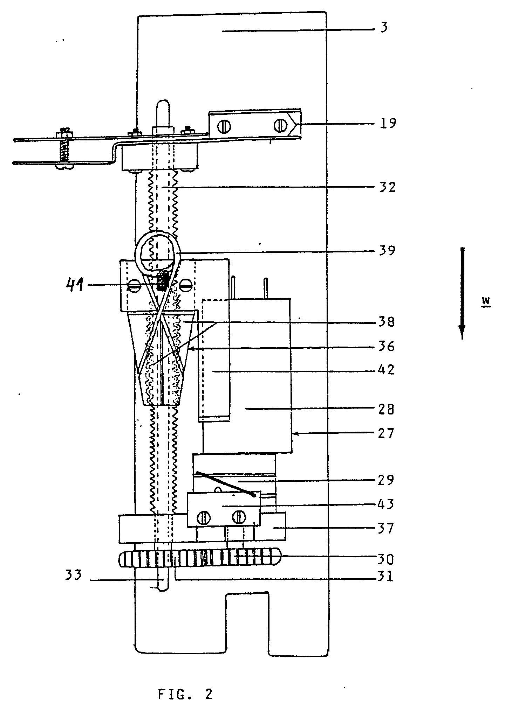

Wie insbesondere Fig. 2 und 3 zeigen, ist zur weiteren Steuerung der Injektionssonde 1 eine zweite Antriebsvorrichtung 27 vorgesehen, die ebenfalls in einem unteren Teil des Gehäuses 2 untergebracht ist. Die Antriebsvorrichtung 27 enthält einen Motor 28, dessen Ausgangswelle über ein zweites Reduktionsgetriebe 29 mit einem Zahnrad 30 verbunden ist, das mit einem zweiten Zahnrad 31 kämmt, an dem eine am Schlitten 3 drehbar gelagerte zweite Gewindestange 32 befestigt ist. Die Drehachsen aller dieser Teile liegen parallel zur Richtung des Doppelpfeils v . Auf der Oberseite des Schlittens 3 ist eine zum Doppelpfeil v parallele Schlitzführung 33 ausgebildet, in der ein Schieber 34 hin- und herverschiebbargelagert ist. Dieser Schieber 34 weist einen seitlich wegragenden Arm 35 auf, der den Kolben 12 der Injektionssonde 1 übergreift und zu dessen Verschiebung dient. Im übrigen ist der Schieber 34 mit einer auf die zweite Gewindestange 32 aufgezogenen Gewindehülse 36 verbunden, so daß er beim Einschalten des Motors 28 über die Zahnräder 30,31 und die Gewindestnge 32 in eine zum Doppelpfeil v parallele Bewegung versetzt wird. Die gesamte zweite Antriebsvorrichtung 27 wird von einem am Schlitten 3 befestigten Lagerblock 37 getragen, so daß sie als Ganzes die Schlittenbewegungen mitmacht.

Der Motor 28 könnte grundsätzlich ebenfalls ein Reversiermotor sein und ähnlich wie der Reversiermotor 15 gesteuert werden. Zur Vereinfachung des Auswechselns bzw. Einlegens der Spritze 11,12 ist der Motor 28 jedoch nur ein in eine Richtung drehender Motor (Pfeil w in Fig. 2), der durch eine zwischen ihn und den Schieber 34 geschaltete Kupplung von diesem entkoppelt werden kann. Die Kupplung wird beispielsweise dadurch geschaffen, daß die Gewindehülse 36 aus zwei Halbschalen 38 zusammengesetzt wird, die durch eine Feder 39 zusammengehalten werden. Zur Betätigung der Kupplung ist auf der Oberseite des Schiebers 34 ein Drehknopf 40 vorgesehen, der auf seiner Rückseite ein die Schlitzführung 33 durchragendes und zwischen den beiden Halbschalen 38 angeordnetes Spreizelement 41 aufweist. Hierdurch ist es möglich, durch Betätigung des Drehknopfs 40 die beiden Halbschalen 38 gegen den Druck der Feder 39 so weit voneinander zu entfernen, daß sie von der Gewindestange 32 entkoppelt sind und der Schieber 34 mit der Hand zwischen seinen Endstellungen hin- und herbewegt werden kann. Dies ermöglicht die Ankopplung des Schiebers 34 an jede beliebige Stelle der Gewindestange 32, um beispielsweise den Arm 34 auf unterschiedlich lange Spritzen einstellen zu können.

An der Gewindehülse 36 ist ein Ansatz 42 befestigt, den ein am Lagerblock 37 befestigter Endschalter 43 zugeordnet ist. Dieser Endschalter 43 legt den Bewegungshub des Schiebers 34 in Richtung des Pfeils w fest der genau dem Weg des Kolbens 12 im Zylinder 11 der Spritze entspricht. Der Endschalter 43 ist dazu in einen Schaltkreis des Motors 28 geschaltet, der außerdem mittels eines weiteren Fußschalters 44 ein- und ausgeschaltet werden kann. Zur Funktionskontrolle des Motors 28 dienen im übrigen ein auf der Oberseite des Gehäuses 2 angeordneter und dieselbe Funktion wie der Fußschalter 44 aufweisender Handschalter 45 und eine Kontrolleuchte 46, die nur bei laufendem Motor 28 aufleuchtet und zusammen mit dem Handschalter 43 ebenfalls in den Schaltkreis des Motors 28 geschaltet ist.

Die Betriebsweise der beschriebenen Vorrichtung ist wie folgt:
Nach dem Festlegen der Ummantelung 6 der Injektionssonde 1 in der Aufnahme 5 und des Bedienungsorgans 8 in der Aufnahme 7 kann eine aufgezogene und mit dem Bedienungsorgan 8 verbundene, z.B. leere Spritze in die Aufnahme 13 eingelegt werden. Sodann wird der Schieber 34 durch Betätigung des Drehknopfs 40 vom Motor 28 entkoppelt und mit der Hand derart verschoben, daß der Arm 35 dem Ende des Kolbens 12 anliegt. Darauf wird der Schieber 34 durch erneute Betätigung des Drehknopfs 40 wieder mit dem Motor 28 gekoppelt. Anschließend werden mit den Handschaltern 25a,b und 45 bzw. nach Anschluß der Fußschalter 23a,b und 44 auch mit diesen die verschiedenen Funktionen der Vorrichtung kontrolliert, wobei die Kontrolleuchten jeweils die zugeordnete Funktion sichtbar machen. Abschließend wird der Schieber 34 wieder zurückgefahren und die leere Spritze gegen eine gefüllte Spritze mit ausgefahrenem Kolben ersetzt.

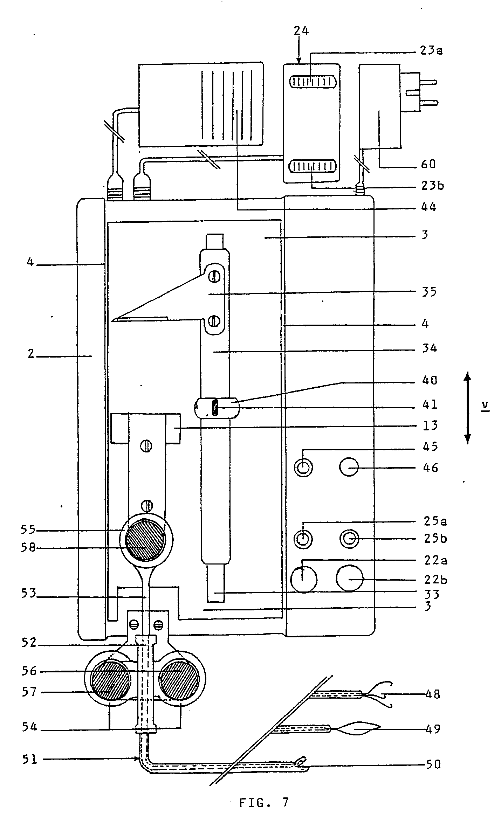

Fig. 7, in der gleiche Teile mit gleichen Bezugszeichen bezeichnet sind, zeigt die Anwendung der beschriebenen Vorrichtung für andere Endoskopieverfahren, beispielsweise solchen, bei denen eine Sonde mit einem anderen medizinischen Instrument, z.B. einem Greifer 48, einer Koagulationsschlinge 49 oder ein Biopsiezange 50, eingesetzt wird. Jede dieser Sonden weist eine Ummantelung 51 auf, in der ein Betätigungselement 52 nach Art eines Bowdenzugs od. dgl. angeordnet ist, dessen einer, aus dem rückwärtigen Ende der Ummantelung 51 herausragender Endabschnitt an einem Bedienungselement 53 befestigt und dessen anderer Endabschnitt in bekannter Weise zur Betätigung desjenigen Instruments, z.B. des Greifers 48, der Koagulationsschlinge 49 oder der Biopsiezange 50, dient, das am vorderen Ende der Sonde beweglich angeordnet ist. Zur Betätigung derartiger Sonden dienen normalerweise zwei relativ zur Ummantelung 51 starr angeordnete Ringe 54 zum Einlegen des Zeige- und Mittelfingers einer Hand und ein am Bedienungselement 53 befestigter Ring 55, in den der Daumen derselben Hand eingelegt wird, um das jeweilige medizinische Instrument zu betätigen. Da auch bei Anwendung derartiger Sonden bisher die Betätigung der Instrumente nur von einer Hilfsperson durchgeführt werden kann, sind die Aufnahmem 5 und 7 der Vorrichtung nach Fig. 1 abnehmbar am Gehäuse 2 bzw. am Schlitten 3 befestigt. Wird für das Endoskopiegerät eine andere als die in Fig. 1 dargestellte Injektionssonde 1 benötigt, können diese Aufnahmen 5 und 7 demontiert und gegen andere Aufnahmen 56,57 und 58 ausgewechselt werden, die beispielsweise dazu geeignet sind, die in Fig. 7 dargestellten Ringe 54,55 klemmend aufzunehmen und damit die Ummantelung 51 am Gehäuse 2 bzw. das Bedienungsorgan 53 am Schlitten 3 festzulegen. Sollte es in diesen Fällen erforderlich sein, den durch die Endschalter 20a,b festgelegten Bewegungshub des Schlittens 3 an die im Einzelfall vorgesehene Sonde anzupassen, ist vorzugsweise wenigstens einer der Endschalter 20a,b an einer leicht zugänglichen Stelle des Gehäuses 2 verschiebbar bzw. einstellbar angeordnet.

Bei dem Reversiermotor 15 und dem Motor 28 handelt es sich vorzugsweise um 12 V-Antriebsmotoren, die über einen aus Sicherheitsgründen außerhalb des Gehäuses 2 befindlichen 220 V-Transformator 60 angetrieben werden.

Fig. 4,5 und 6 zeigen schematisch die Verschaltung der Antriebseinheiten 15,28, der Kontrolleuchten 22a,b,46, der Bedienungsschalter 23a,b,25a,b,44 und 45 und der Endschalter 20a,b, 21a,b und 43 in entsprechenden elektrischen Stromkreisen.

In Fig. 4 befinden sich die Bedienungsschalter 23a,b und 25a,b im unbetätigten Zustand und die beiden Endschalter 20a,b in der geschlossenen Stellung, so daß beide Anschlüsse des Reversiermotors 15 mit der ⊕-Leitung des Transformators 60 verbunden sind. Der Reversiermotor 15 steht daher still. Bei Betätigung einer der Schalter 23a,b bzw. 25a,b wird dagegen einer der Anschlüsse des Reversiermotors 15 mit der ⊖-Leitung des Transformators 60 verbunden, so daß sich der Reversiermotor 15 in der einen oder anderen Richtung dreht. Wird beim Erreichen einer Endstellung der zugehörige Endschalter 20a,b betätigt, hat dies wiederum eine Verbindung des entsprechenden Anschlusses mit der ⊕-Leitung zur Folge, so daß der Reversiermotor 15 in der Endstellung stehen bleibt, bis eine Betätigung in der entgegengesetzten Richtung erfolgt, wodurch der betreffende Endschalter 20a,b in die Lage nach Fig. 4 zurückkehrt und dadurch die Ausgangslage nach Fig. 4 wiederhergestellt ist. Fig. 5 zeigt, daß gleichzeitig mit der Umschaltung der Endschalter 21a,b jeweils die die Kontrolleuchten 22a,b enthaltenden Strompfade zwischen der ⊕- und ⊖-Leitung geschlossen werden.

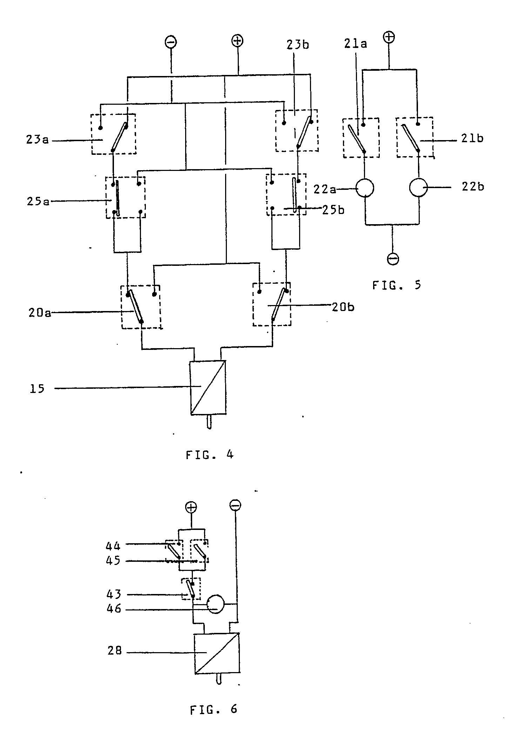

Gemäß Fig. 6 sind die beiden Anschlüsse des Motors 28 mit der ⊕-Leitung bzw. ⊖-Leitung verbunden, wobei in den einen Zweig der Endschalter 43 und eine den Fußschalter 44 und den Handschalter 45 enthaltende Parallelschaltung geschaltet sind. Bei Betätigung eines der Schalter 44,45 wird somit der Motor 28 erregt, während gleichzeitig die Kontrolleuchte 46 leuchtet. Öffnet der Endschalter 43, so hat dies ein Erlöschen der Kontrolleuchte 46 zur Folge.

Die Erfindung ist nicht auf die beschriebenen Ausführungsbeispiele beschränkt, die sich auf vielfache Weise abwandeln lassen. Dies gilt insbesondere für die in den Ausführungsbeispielen gewählte spezielle Ausbildung der verschiedenen Aufnahmen, Antriebseinrichtungen und deren Steuerungen.

**Ansprüche**

1. Vorrichtung mit einer eine Ummantelung aufweisenden Sonde eines Endoskopiegeräts und Mitteln zur Steuerung der Sonde, wobei die Sonde an ihrem einen Ende ein bewegliches medizinisches Instrument, an ihrem anderen Ende ein Bedienungsorgan und außerdem ein die Ummantelung durchragendes, mit dem Bedienungsorgan und dem Instrument verbundenes Betätigungselement aufweist, dadurch gekennzeichnet, daß die Mittel zur Steuerung der Sonde ein Gehäuse (2), eine an diesem befestigte erste , die Ummantelung (6) festlegende Aufnahme (5,56,57), einen im Gehäuse (2) in entgegengesetzte Richtungen verschiebbar gelagerten Schlitten (3), eine am Schlitten (3) befestigte zweite, das Bedienungsorgan (8,53) festlegende Aufnahme (7,58), eine im Gehäuse (2) gelagerte erste Antriebseinrichtung (14) zum selbsttätigen Verschieben des Schlittens (3) in beide Richtungen und eine Schalteinrichtung (23a,b; 25a,b) zum wahleisen Einschalten der Antriebseinrichtung (14) in der einen oder anderen Richtung aufweisen.

2. Vorrichtung nach Anspruch 1, dadurch gekennzeichnet, daß sie mit einer Injektionssonde, deren Bedienungsorgan mit einem Anschluß für eine medizinische, einen Zylinder und einen Kolben aufweisende Spritze versehen ist und daß an dem Schlitten (3) eine dritte, den Zylinder (11) der Spritze festlegende Aufnahme (13) und eine zweite Antriebseinrichtung (27) zur Betätigung des Kolbens (12) der Spritze befestigt sind, wobei die zweite Antriebseinrichtung (27) einen verschiebbar am Schlitten gelagerten Schieber (34) und einen Schalter (44,45) zum wahlweisen Einschalten der Antriebseinrichtung (27) aufweist.

3. Vorrichtung nach Anspruch 1 oder 2, dadurch gekennzeichnet, daß die Schaltvorrichtung einen mit dem Fuß zu betätigenden Doppelschalter (24) aufweist.

4. Vorrichtung nach wenigstens einem der Ansprüche 1 bis 3, dadurch gekennzeichnet, daß der Schalter (44) ein mit dem Fuß zu betätigender Schalter ist.

5. Vorrichtung nach wenigstens einem der Ansprüche 1 bis 4, dadurch gekennzeichnet, daß die erste Antriebseinrichtung (14) eine drehbar im Gehäuse (2) gelagerte erste Gewindestange (17), einen Reversiermotor (15) mit einer mit der ersten Gewindestange (17) verbundenen Ausgangswelle und eine am Schlitten (3) befestigte, von der ersten Gewindestange (17) durchragte Gewindemutter (18) aufweist.

6. Vorrichtung nach wenigstens einem der Ansprüche 1 bis 5, dadurch gekennzeichnet, daß die zweite Antriebseinrichtung (27) einen Motor (28) zur Verschiebung des Schiebers (34) und eine zwischen diesen und den Motor (28) geschaltete Kupplung aufweist.

7. Vorrichtung nach Anspruch 6, dadurch gekennzeichnet, daß die zweite Antriebseinrichtung (27) eine drehbar am Schlitten (3) gelagerte zweite Gewindestange (32) aufweist, die mit der Ausgangswelle des Motors (28) verbunden ist, und daß die Kupplung aus einer auf die zweite Gewindestange (32) aufgezogenen Gewindehülse (36) besteht, die zwei durch eine Feder (39) zusammengehaltene Halbschalen (38) und einen am Schieber (35) gelagerten Drehknopf (40) aufweist, der ein zwischen die beiden Halbschalen (38) ragendes Spreizelement (41) trägt.

8. Vorrichtung nach wenigstens einem der Ansprüche 5 bis 7, dadurch gekennzeichnet, daß die Gewindemutter (18) einen radial abstehenden Arm (19) aufweist, der zwischen zwei am Gehäuse befestigten Endschaltern (20a,b) angeordnet ist, die einen dem maximal zulässigen Bewegungshub des Schlittens (3) in den beiden entgegengesetzten Richtungen entsprechenden Abstand voneinander aufweisen.

9. Vorrichtung nach Anspruch 8, dadurch gekennzeichnet, daß der Arm (19) zwischen zwei weiteren am Gehäuse (2) befestigten Endschaltern (21a,b) angeordnet ist, die mit die Endstellungen des Schlittens (3) anzeigenden Kontrolleuchten (22a,b) verbunden sind.

10. Vorrichtung nach wenigstens einem der Ansprüche 2 bis 9, dadurch gekennzeichnet, daß die zweite Antriebseinrichtung (27) einen an der Gewindehülse (36) befestigten Ansatz (42) aufweist, dem ein das Ende des Bewegungshubs des Schiebers (34) begrenzender, am Schlitten (3) befestigter Endschalter (43) zugeordnet ist.

11. Vorrichtung nach wenigstens einem der Ansprüche 5 bis 10, dadurch gekennzeichnet, daß die beiden Gewindestangen (17,32) parallel zueinander angeordnet sind.

## Claims

1. Device with a sheathed probe of an endoscopy apparatus and means for controlling the probe, wherein the probe comprises a movable medical instrument at its one end, a control member at its other end and moreover an actuating element extending through the sheath and connected to the control member and to the instrument, characterized in that the means for controlling the probe comprise a housing (2), a first receptacle (5, 56, 57) fixing the sheath (6) and fixed on the housing, a slide (3) mounted in the housing (2) to slide in opposite directions, a second receptacle (7, 58) fixing the control member (8, 53) and fixed on the slide (3), a first drive device (14) mounted in the housing (2) for automatic displacement of the slide (3) in both directions and a circuit device (23a,b; 25a,b) for selectively switching on the drive device (14) in the one or the other direction.

2. Device according to claim 1, characterized in that it is provided with an injection probe, whose control member is provided with a connection for a medical syringe having a cylinder and a piston, and in that there are fixed on the slide (3) a third receptacle (13) fixing the cylinder (11) of the syringe and a second drive device (27) for actuation of the piston (12) of the syringe, wherein the second drive device (27) comprises a slider (34) slidably mounted on the slide and a switch (44, 45) for selectively switching on the drive device (27).

3. Device according to claim 1 or 2, characterized in that the circuit device comprises a foot operated double switch (24).

4. Device according to at least one of claims 1 to 3, characterized in that the switch (44) is a foot operated switch.

5. Device according to at least one of claims 1 to 4, characterized in that the first drive device (14) comprises a first threaded rod (17) rotatably mounted in the housing (2), a reversible motor (15) with an output shaft coupled to the first threaded rod (17) and a nut (18) fixed on the slide (3) and through which the first threaded rod (17) passes.

6. Device according to at least one of claims 1 to 5, characterized in that the second drive device (27) comprises a motor (28) for displacing the slider (34) and a clutch coupled between this and the motor (28).

7. Device according to claim 6, characterized in that the second drive device (27) comprises a second threaded rod (32) rotatably mounted in the slide (3), which rod is coupled to the output shaft of the motor (28), and in that the clutch consists of a threaded sleeve (36) fitted on the second threaded rod (32) and which comprises two half shells (38) held together by a spring (39) and a rotary knob (40) mounted on the slider (35), which carries a spreading element (41 projecting between the two half shells (38).

8. Device according to at least one of claims 5 to 7, characterized in that the nut (18) has a radially projecting arm (19), which is arranged between two limit switches (20a,b) fixed on the housing (2), which switches have a spacing from one another corresponding to a maximum permitted stroke of the slide (3) in the two opposite directions.

9. Device according to claim 8, characterized in that the arm (19) is arranged between two further limit switches (21a,b) fixed on the housing (2), which switches are connected to control lights (22a,b) indicating the end positions of the slide (3).

10. Device according to at least one of claims 2 to 9, characterized in that the second drive device (27) has a projection (42) fixed on the threaded sleeve (36), with which is associated a limit switch (43) fixed on the slide (3) and delimiting the end of the stroke of the slider (34).

11. Device according to at least one of claims 5 to 10, characterized in that the two threaded rods (17, 32) are arranged parallel to one another.

**Revendications**

1. Dispositif comprenant une sonde d'un appareil d'endoscopie équipée d'une gaine, et des moyens pour la commande de la sonde, la sonde présentant à l'une de ses extrémités un instrument médical mobile, à l'autre extrémité un organe de manipulation, de même qu'un élément d'actionnement qui traverse la gaine et est relié à l'organe de manipulation et à l'instrument, **caractérisé en ce** que les moyens pour la commande de la sonde comprennent un boîtier (2), un premier logement (5, 56, 57) fixé sur ledit boîtier et immobilisant la gaine (6), un chariot (3) monté dans le boîtier (2) et déplaçable dans des directions opposées, un second logement (7, 58) fixé sur le chariot (3) et immobilisant l'organe de manipulation (8, 53), un premier dispositif d'entraînement (14) monté dans le boîtier (2) pour le déplacement automatique du chariot (3) dans les deux directions, et un dispositif de commutation (23a, b; 25a, b) pour connecter le dispositif d'entraînement (14), au choix, dans l'une ou l'autre direction.

2. Dispositif selon la revendication 1, caractérisé en ce qu'il comprend une sonde d'injection dont l'organe de manipulation est muni d'un raccord pour une seringue médicale pourvue d'un cylindre et d'un piston; et que sur le chariot (3) sont fixés un troisième logement (13) immobilisant le cylindre (11) de la seringue et un second dispositif d'entraînement (27) pour l'actionnement du piston (12) de la seringue, le second dispositif d'entraînement (27) comprenant un coulisseau (34) monté de manière mobile sur le chariot, et un commutateur (44, 45) pour connecter, au choix, le dispositif d'entraînement (27).

3. Dispositif selon l'une des revendications 1 ou 2, caractérisé en ce que le dispositif de commutation comprend un commutateur double (24) à actionner avec le pied.

4. Dispositif selon l'une des revendications 1 à 3, caractérisé en ce que le commutateur (44) est un commutateur à pédale.

5. Dispositif selon l'une des revendications 1 à 4, caractérisé en ce que le premier dispositif d'entraînement (14) comprend une première tige filetée (17) montée de manière tournante dans le boîtier (2), un moteur réversible (15) avec un arbre de sortie couplé avec la première tige filetée (17), et un écrou (18) fixé sur le chariot (3) et traversé par la première tige filetée (17),

6. Dispositif selon l'une des revendications 1 à 5, caractérisé en ce que le second dispositif d'entraînement (27) comprend un moteur (28) pour le déplacement du coulisseau (34) et un embrayage intercalé entre celui-ci et le moteur (28).

7. Dispositif selon la revendication 6, caractérisé en ce que le second dispositif d'entraînement (27) comprend une seconde tige filetée (32) montée de manière tournante sur le chariot (3) et couplée avec l'arbre de sortie du moteur (28); et que l'embrayage se compose d'une douille filetée (36) engagée sur la seconde tige filetée (32), laquelle douille filetée comprend deux demi-coques (38) maintenues ensemble par un ressort (39) ainsi qu'un bouton tournant (40) monté sur le coulisseau (35) et portant un élément d'écartement (41) engagé entre les deux demi-coques (38).

8. Dispositif selon l'une des revendications 5 à 7, caractérisé en ce que l'écrou (18) comporte un bras (19) qui s'écarte radialement et est disposé entre deux interrupteurs de fin de course (20a, b) fixés sur le boîtier à une distance correspondant à la course maximale admissible du chariot (3) dans les deux directions opposées.

9. Dispositif selon la revendication 8, caractérisé en ce que le bras (19) est disposé entre deux interrupteurs de fin de course supplémentaires (21a, b) fixés sur le boîtier (2) et reliés à des lampes témoin (22a, b) indiquant les positions de fin de course du chariot (3).

10. Dispositif selon l'une des revendications 2 à 9, caractérisé en ce que le second dispositif d'entraînement (27) comprend un embout (42) fixé sur la douille filetée (36) et auquel est associé un interrupteur de fin de course (43) monté sur le chariot (3) et délimitant l'extrémité de la course du coulisseau (34).

13 EP 0 212 225 B1 14

**11.** Dispositif selon l'une des revendications 5 à 10, caractérisé en ce que les deux tiges filetées (17, 32) sont disposées parallèlement l'une par rapport à l'autre.

8

FIG. 1

FIG. 2

FIG. 3

FIG. 4

FIG. 5

FIG. 6

FIG. 7